# FASCICULE DE BREVET EUROPEEN

(11) **EP 2 324 816 B1**
(45) Date de publication et mention de la délivrance du brevet: **02.01.2019**
(21) Numéro de dépôt: 10189851.8
(22) Date de dépôt: 03.11.2010
(51) Int. Cl.: A61K 8/31, A61K 8/34, A61Q 13/00, D06M 13/00

(54) **Composition parfumante aqueuse comprenant au moins un alcane lineaire volatil ; procede de parfumage**
Parfümierende wässrige Zusammensetzung, die mindestens ein flüchtiges lineares Alkan enthält, Parfümierungsverfahren
Aqueous flavouring composition including at least one volatile linear alkane; flavouring method

(30) Priorité: 19.11.2009 FR 0958193
(43) Date de publication de la demande: 25.05.2011
(73) Titulaire: L'Oréal, 75008 Paris (FR)
(72) Inventeur: Grandjon, Vincent, 95200 Sarcelles (FR); Noel-Poquet, Christine, 91320 Wissous (FR)
(74) Mandataire: Miszputen, Laurent

(56) Documents cités:
- WO-A1-02/32390
- WO-A1-2005/004828
- WO-A1-2008/054067
- WO-A2-2008/155059
- FR-A1- 2 777 180
- GB-A- 2 165 150
- US-A- 1 912 799
- US-A- 2 995 278
- US-A1- 2009 209 449
- "Composition huileuse volatile", RESEARCH DISCLOSURE, MASON PUBLICATIONS, HAMPSHIRE, GB, vol. 548, no. 16, 1 décembre 2009 (2009-12-01), page 1220, XP007139494, ISSN: 0374-4353

## Description

La présente invention concerne un procédé de parfumage des matières kératiniques humaines ou d'un vêtement qui consiste à appliquer sur lesdites matières kératiniques ou ledit vêtement une composition parfumante aqueuse sous forme d'émulsion huile-dans-eau comprenant dans un milieu cosmétiquement acceptable a) de 30 à 80% en poids d'eau par rapport au poids total de la composition; b) au moins 2% en poids d'une substance parfumante par rapport au poids total de la composition; c) au moins un alcane linéaire volatil ou un mélange d'alcanes linéaires volatils, ledit ou lesdits alcanes volatils comprenant de 7 à 14 atomes de carbone.

On sait qu'un parfum est l'association de différentes substances odorantes qui s'évaporent à des périodes différentes. Chaque parfum présente ce que l'on appelle une « note de tête » qui est l'odeur diffusant en premier lors de l'application du parfum ou lors de l'ouverture du récipient le contenant, une « note de coeur ou corps » qui correspond au parfum complet (émission pendant quelques heures après la « note de tête ») et une « note de fond » qui est l'odeur la plus persistante (émission pendant plusieurs heures après la « note de coeur »). La persistance de la note de fond correspond à la rémanence du parfum.

L'être humain a de tout temps cherché à se parfumer et à parfumer les objets qui l'entourent ou les lieux dans lesquels il se trouve, et ceci, aussi bien pour masquer des odeurs fortes et/ou désagréables que pour donner une bonne odeur.

Il est courant d'incorporer du parfum dans un certain nombre de produits ou compositions, en particulier cosmétiques et dermatologiques telles que des eaux fraîches, eaux de toilette, des eaux de parfum, des élixirs ou extraits de parfum, des lotions après rasage, des eaux de soin, des lotions bi-phasiques.

Les consommateurs recherchent et apprécient particulièrement les produits cosmétiques parfumés aqueux. La présence d'une phase aqueuse apporte à ces produits de la fraîcheur. Et la sensation de fraîcheur est d'autant plus grande que la teneur en eau est élevée.Le parfum quant à lui est jugé comme un élément de plaisir ; l'homme de métier fait en sorte de les faire exhaler afin de répondre aux attentes des utilisateurs.

Les concentrés parfumant sont composés de matières premières odorantes dont les pressions de vapeur sont faibles à température ambiante (25°C) et généralement liquides mais parfois solides donc peu volatils.

Les compositions cosmétiques parfumées à fortes teneurs en eau (au moins 30% en poids) sont quant à elle habituellement présentées sous forme de laits, de crèmes, de baumes, d'émulsions fluides, de gel-crèmes, de pâtes, de lotions multi-phasiques. Toutes ces préparations cosmétiques physiologiquement acceptables contiennent des émollients et/ou des huiles minérales et/ou végétales, et/ou des silicones, et/ou des alcools de Guerbet et/ou des esters ou éthers d'acides gras.

Ces composés sont de bons agents émollients et adoucissants bien connus permettant d'obtenir des supports parfumés mais il s'avère que ces composés sont connus pour fixer les odeurs.

Ces composés sont généralement non volatiles ou très faiblement volatiles. En effet les composés d'origine végétale comme les esters, les éthers, les huiles végétales ont la fâcheuse tendance à fixer et retenir les parfums notamment car elles ne sont pas ou très peu volatiles ; les parfums sont comme « étouffés, aplatis » ; comme liés aux huiles, et perdent par conséquence leurs caractères diffusants.

Le parfumage en est donc fortement affecté ; le parfum reste lié au support, en effet le parfum s'évapore beaucoup moins vite, il diffuse beaucoup moins, il ne s'exhale plus. Ce qui va à l'encontre de l'attente des utilisateurs qui attentent d'un produit cosmétique parfumé qu'il « sente ».

Il est bien évidemment possible d'augmenter la concentration en parfum de la préparation cosmétique mais il s'avère que les parfums en grande quantité ont un potentiel irritant pour la peau et sont des composés coûteux voir très coûteux.

Il est possible d'utiliser des composés halogénés efficaces comme les perfluoroethers mais ces composés sont onéreux, peu abondants et très difficile à formuler.

On peut également utiliser un alcanol en C₁-C₅ comme l'éthanol pour permettre d'améliorer la diffusion des parfums. Cependant, ils présentent l'inconvénient d'altérer les caractéristiques olfactives des ingrédients parfumants, en raison non seulement de leur odeur puissante mais aussi de leur capacité à réagir, en présence d'eau, avec les ingrédients parfumants et à modifier ainsi leur odeur et/ou leur couleur. Enfin, ils ont un potentiel irritant et ont tendance à dessécher la peau. Ils peuvent être une source de picotements lorsqu'ils sont appliqués sur une peau sensible ou lésée, notamment après le rasage. De plus, en raison de leur faible point éclair (éthanol avec un point éclair de13°C) et de l'inflammabilité, leur incorporation dans des formulations comprenant une phase grasse que l'on chauffe à des températures supérieures à 60°C nécessite des contraintes industrielles pour des raisons de sécurité.

Il est également possible d'utiliser des huiles de silicones cycliques comme le cyclomethicone, le cyclopentasiloxane ou le cyclohexasiloxane cependant ces composés sont peu émollients pour la peau. Il est aussi possible d'utiliser des hydrocarbures volatils comme isohexadécane ou l'isododécane mais leurs origine pétrochimique est de nos jours très critiquée. Cependant, ces solutions ne se révèlent pas toujours satisfaisantes, notamment lors de l'utilisation de matières premières naturelles ou d'origine naturelle pour formuler les compositions cosmétiques.

Or, parallèlement, les consommatrices sont de plus en plus à la recherche de produits cosmétiques formés, en tout ou partie, de constituants végétaux ou d'origine végétale.

Il subsiste le besoin de rechercher de nouvelles formulations parfumantes aqueuses à fortes teneurs en eau dans lesquelles
- les substances parfumantes soient correctement solubilisées dans un support formé en tout ou partie de constituants végétaux ou d'origine végétale
- la diffusion du parfum est améliorée
- pouvant être facilement stockées et fabriquées sans les contraintes imposées par les solvants volatils de l'art antérieur et ce sans les inconvénients énoncés précédemment

La demanderesse a découvert de manière surprenante qu'on pouvait atteindre cet objectif avec une composition parfumante aqueuse comprenant dans un milieu cosmétiquement acceptable
a) au moins 5% en poids d'eau par rapport au poids total de la composition ;
b) au moins 2% en poids d'une substance parfumante ;
c) au moins un alcane linéaire volatil ou un mélange d'alcanes linéaires volatils. la teneur en eau variant de 30 à 80% par rapport au poids total de la composition Cette découverte est à la base de l'invention.

La présente invention concerne un procédé de parfumage des matières kératiniques humaines ou d'un vêtement qui consiste à appliquer sur lesdites matières kératiniques ou ledit vêtement une composition parfumante aqueuse sous forme d'émulsion huile-dans-eau comprenant dans un milieu cosmétiquement acceptable a) de 30 à 80% en poids d'eau par rapport au poids total de la composition; b) au moins 2% en poids d'une substance parfumante par rapport au poids total de la composition; c) au moins un alcane linéaire volatil ou un mélange d'alcanes linéaires volatils, ledit ou lesdits alcanes volatils comprenant de 7 à 14 atomes de carbone.

Par « substance parfumante», on entend tout parfum toute matière première odorante ou arôme susceptible de dégager une odeur agréable.

On entend par « matières kératiniques humaines », la peau (visage, corps, les lèvres), le cuir chevelu, les cheveux, les cils, les sourcils, les ongles, les muqueuses.

On entend par milieu cosmétiquement acceptable, un milieu non toxique et susceptible d'être appliqué sur la peau, les lèvres, les ongles, les cils, les sourcils, le cuir chevelu, les cheveux et les muqueuses.

Par « solvant volatil » ou "huile volatile", on entend un solvant ou une huile (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm de Hg), et de préférence allant de 1,3 Pa à 13 000 Pa (0,01 à 100 mm de Hg), et préférentiellement allant de 1,3 Pa à 1300 Pa (0,01 à 10 mm de Hg).

### ALCANES LINEAIRES VOLATILS

La composition selon l'invention contient un ou plusieurs alcane(s) linéaire(s) volatil(s). Par « un ou plusieurs alcane(s) linéaire(s) volatil(s) », on entend indifféremment « une ou plusieurs huile alcane(s) linéaire(s) volatile(s) ».

Un alcane linéaire volatil convenant à l'invention est liquide à température ambiante (environ 25 °C) et à la pression atmosphérique (760 mm Hg).

Par « alcane linéaire volatil » convenant à l'invention, on entend un alcane linéaire cosmétique, susceptible de s'évaporer au contact de la peau en moins d'une heure, à température ambiante (25°C) et pression atmosphérique (760 mm Hg, c'est-à-dire 101 325 Pa), liquide à température ambiante, ayant notamment une vitesse d'évaporation allant de 0,01 à 15 mg/cm²/min, à température ambiante (25°C) et pression atmosphérique (760 mm Hg).

De façon préférée, les « alcanes linéaires volatils » convenant à l'invention présentent une vitesse d'évaporation allant de 0,01 à 3,5 mg/cm²/min, à température ambiante (25°C) et pression atmosphérique (760 mm Hg).

De façon préférée, les « alcanes linéaires volatils » convenant à l'invention présentent une vitesse d'évaporation allant de 0,01 à 1,5 mg/cm²/min, à température ambiante (25°C) et pression atmosphérique (760 mm Hg).

De façon plus préférée, les « alcanes linéaires volatils » convenant à l'invention présentent une vitesse d'évaporation allant de 0,01 à 0,8 mg/cm²/min, à température ambiante (25°C) et pression atmosphérique (760 mm Hg).

De façon encore préférée, les « alcanes linéaires volatils » convenant à l'invention présentent une vitesse d'évaporation allant de 0,01 à 0,3 mg/cm²/min, à température ambiante (25°C) et pression atmosphérique (760 mm Hg).

De façon encore préférée, les « alcanes linéaires volatils » convenant à l'invention présentent une vitesse d'évaporation allant de 0,01 à 0,12 mg/cm²/min, à température ambiante (25°C) et pression atmosphérique (760 mm Hg).

La vitesse d'évaporation d'un alcane volatil conforme à l'invention (et plus généralement d'un solvant volatil) peut être notamment évaluée au moyen du protocole décrit dans WO 06/013413, et plus particulièrement au moyen du protocole décrit ci-après.

On introduit dans un cristallisoir (diamètre : 7 cm) placé sur une balance se trouvant dans une enceinte d'environ 0,3 m³ régulée en température (25 °C) et en hygrométrie (humidité relative 50 %) 15 g de solvant hydrocarboné volatil.

On laisse le liquide s'évaporer librement, sans l'agiter, en assurant une ventilation par un ventilateur (PAPST-MOTOREN, référence 8550 N, tournant à 2700 tours/minute) disposé en position verticale au-dessus du cristallisoir contenant le solvant hydrocarboné volatil, les pales étant dirigées vers le cristallisoir, à une distance de 20 cm par rapport au fond du cristallisoir.

On mesure à intervalles de temps réguliers la masse de solvant hydrocarboné volatil restante dans le cristallisoir.

On obtient alors le profil d'évaporation du solvant en traçant la courbe de la quantité de produit évaporé (en mg/cm²) en fonction du temps (en min). Puis on calcule la vitesse d'évaporation qui correspond à la tangente à l'origine de la courbe obtenue. Les vitesses d'évaporation sont exprimées en mg de solvant volatil évaporé par unité de surface (cm²) et par unité de temps (minute).

Selon un mode de réalisation préféré, les « alcanes linéaires volatils » convenant à l'invention ont une pression de vapeur (appelée également pression de vapeur saturante) non nulle, à température ambiante, en particulier une pression de vapeur allant de 0,3 Pa à 6000 Pa.

De façon préférée, les « alcanes linéaires volatils » convenant à l'invention ont une pression de vapeur allant de 0,3 à 2000 Pa, à température ambiante (25°C).

De façon préférée, les « alcanes linéaires volatils » convenant à l'invention ont une pression de vapeur allant de 0,3 à 1000 Pa, à température ambiante (25°C)

De façon plus préférée, les « alcanes linéaires volatils » convenant à l'invention ont une pression de vapeur allant de 0,4 à 600 Pa, à température ambiante (25°C).

De façon préférée, les « alcanes linéaires volatils » convenant à l'invention ont une pression de vapeur allant de 1 à 200 Pa, à température ambiante (25°C).

De façon encore préférée, les « alcanes linéaires volatils » convenant à l'invention ont une pression de vapeur allant de 3 à 60 Pa, à température ambiante (25°C).

Selon un mode de réalisation, un alcane linéaire volatil convenant à l'invention peut présenter un point éclair compris dans l'intervalle variant de 30 à 120 °C, et plus particulièrement de 40 à 100 °C. Le point éclair est en particulier mesuré selon la Norme iso 3679.

Un alcane convenant à l'invention est un alcane linéaire volatil comprenant de 7 à 14 atomes de carbone.

De façon préférée, les « alcanes linéaires volatils » convenant à l'invention comprennent de 8 à 14 atomes de carbone.

De façon préférée, les « alcanes linéaires volatils » convenant à l'invention comprennent de 9 à 14 atomes de carbone.

De façon préférée, les « alcanes linéaires volatils » convenant à l'invention comprennent de 10 à 14 atomes de carbone.

De façon préférée, les « alcanes linéaires volatils » convenant à l'invention comprennent de 11 à 14 atomes de carbone.

Selon un mode de réalisation avantageux, les « alcanes linéaires volatils » convenant à l'invention présentent une vitesse d'évaporation, telle que définie plus haut, allant de 0,01 à 3,5 mg/cm²/min, à température ambiante (25°C) et pression atmosphérique (760 mm Hg), et comprennent de 8 à 14 atomes de carbone.

Un alcane linéaire volatil convenant à l'invention peut être avantageusement d'origine végétale.

De préférence, l'alcane linéaire volatil ou le mélange d'alcanes linéaires volatils présent dans la composition selon l'invention comprend au moins un isotope ¹⁴C du carbone (carbone 14), en particulier l'isotope ¹⁴C peut être présent en un ratio ¹⁴C / ¹²C supérieur ou égal à 1.10⁻¹⁶, de préférence supérieur ou égal à 1.10⁻¹⁵, de préférence encore supérieur ou égal 7,5.10⁻¹⁴, et mieux supérieur ou égal 1,5.10⁻¹³. De préférence, le ratio ¹⁴C / ¹²C va de 6.10⁻¹³ à 1,2.10⁻¹².

La quantité de d'isotopes ¹⁴C dans l'alcane linéaire volatil ou le mélange d'alcanes linéaires volatils peut être déterminée par des méthodes connues de l'homme du métier telles que la méthode de comptage de Libby, la spectrométrie à scintillation liquide ou encore la spectrométrie de masse à accélération (Accelerator Mass Spectrometry).

Un tel alcane peut être obtenu, directement ou en plusieurs étapes, à partir d'une matière première végétale comme une huile, un beurre, une cire, etc.

A titre d'exemple d'alcanes convenant à l'invention, on peut mentionner les alcanes décrits dans les demandes de brevets de la société Cognis WO 2007/068371, ou WO2008/155059 (mélanges d'alcanes distincts et différant d'au moins un carbone). Ces alcanes sont obtenus à partir d'alcools gras, eux-mêmes obtenus à partir d'huile de coprah ou de palme.

A titre d'exemple d'alcanes linéaires convenant à l'invention, on peut citer le n- heptane (C₇), le n-octane (C₈), le n-nonane (C₉), le n-décane (C₁₀), le n-undécane (C₁₁), le n-dodécane (C₁₂), le n-tridécane (C₁₃), le n-tétradecane (C₁₄), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges.

Selon un mode préféré, on peut citer les mélanges de n-undécane (C₁₁) et de n-tridécane (C₁₃) obtenus aux exemples 1 et 2 de la demande WO2008/155059 de la Société Cognis.

On peut également citer le n-dodécane (C₁₂) et le n-tétradécane (C₁₄) vendus par Sasol respectivement sous les références PARAFOL 12-97 et PARAFOL 14-97, ainsi que leurs mélanges.

On pourra utiliser l'alcane linéaire volatil seul.

On pourra alternativement ou préférentiellement utiliser un mélange d'au moins deux alcanes linéaires liquides volatils distincts, différant entre eux d'un nombre de carbone n d'au moins 1, en particulier différant entre eux d'un nombre de carbone de 1 ou de 2.

Selon un premier mode de réalisation, on utilise un mélange d'au moins deux alcanes linéaires volatils distincts comportant de 10 à 14 atomes de carbone et différant entre eux d'un nombre de carbone d'au moins 1. A titre d'exemples, on peut citer notamment les mélanges d'alcanes linéaires volatils C₁₀/C₁₁, C₁₁/C₁₂, ou C₁₂/C₁₃.

Selon un autre mode de réalisation, on utilise un mélange d'au moins deux alcanes linéaires volatils distincts comportant de 10 à 14 atomes de carbone et différant entre eux d'un nombre de carbone d'au moins 2. A titre d'exemples, on peut citer notamment les mélanges d'alcanes linéaires volatils C₁₀/C₁₂, ou C₁₂/C₁₄, pour un nombre de carbone n pair et le mélange C₁₁/C₁₃ pour un nombre de carbone n impair.

Selon un mode préféré, on utilise un mélange d'au moins deux alcanes linéaires volatils comportant de 10 à 14 atomes de carbone distincts et différant entre eux d'un nombre de carbone d'au moins 2, et en particulier un mélange d'alcanes linéaires volatils C₁₁/C₁₃ ou un mélange d'alcanes linéaires volatils C₁₂/C₁₄.

D'autres mélanges associant plus de 2 alcanes linéaires volatils selon l'invention, tels que par exemple un mélange d'au moins 3 alcanes linéaires volatils comportant de 7 à 14 atomes de carbone distincts et différant entre eux d'un nombre de carbone d'au moins 1, font également partie de l'invention, mais les mélanges de 2 alcanes linéaires volatils selon l'invention sont préférés (mélanges binaires), lesdits 2 alcanes linéaires volatils représentant de préférence plus de 95% et mieux plus de 99% en poids de la teneur totale en alcanes linéaires volatils dans le mélange. Selon un mode particulier de l'invention, dans un mélange d'alcanes linéaires volatils, l'alcane linéaire volatil ayant le nombre de carbone le plus petit est majoritaire dans le mélange.

Selon un autre mode de l'invention, on utilise un mélange d'alcanes linéaires volatils dans lequel l'alcane linéaire volatil ayant le nombre de carbone le plus grand est majoritaire dans le mélange.

A titre d'exemples de mélanges convenant à l'invention, on peut citer notamment les mélanges suivants :
- de 50 à 90% en poids, de préférence de 55 à 80% en poids, préférentiellement encore de 60 à 75% en poids d'alcane linéaire volatil liquide en Cₙ avec n allant de 7 à 14
- de 10 à 50% en poids, de préférence de 20 à 45% en poids, de préférence de 24 à 40% en poids, d'alcane linéaire volatil liquide en Cₙ₊ₓ avec x supérieur ou égal à 1, de préférence x=1 ou x=2, avec n+x compris entre 10 et 14, par rapport au poids total des alcanes dans ledit mélange.

En particulier, ledit mélange d'alcanes selon l'invention contient :
- moins de 2% en poids, de préférence moins de 1% en poids d'hydrocarbures ramifiés,
- et/ou moins de 2% en poids, de préférence moins de 1% en poids d'hydrocarbures aromatiques,
- et/ou moins de 2% en poids, de préférence moins de 1% en poids et préférentiellement moins de 0,1% en poids d'hydrocarbures insaturés dans le mélange.

Plus particulièrement, un alcane linéaire volatil convenant à l'invention peut être mis en oeuvre sous la forme d'un mélange n-undécane/n-tridécane.

En particulier, on utilisera un mélange d'alcanes linéaires volatils comprenant :
- de 55 à 80% en poids, de préférence de 60 à 75% en poids d'alcane linéaire volatil liquide en C₁₁ (n-undécane)
- de 20 à 45% en poids, de préférence de 24 à 40% en poids d'alcane linéaire volatil liquide en C₁₃ (n-tridécane)
par rapport au poids total des alcanes dans ledit mélange.

Selon un mode de réalisation particulier, le mélange d'alcanes est un mélange n-undécane/n-tridécane. En particulier un tel mélange peut être obtenu selon l'exemple 1 ou l'exemple 2 du WO 2008/155059.

Selon un autre mode de réalisation particulier, on utilise le n-dodécane vendu sous la référence PARAFOL 12-97 par SASOL.

Selon un autre mode de réalisation particulier, on utilise le n-tétradécane vendu sous la référence PARAFOL 14-97 par SASOL.

Selon encore un autre mode de réalisation, on utilise un mélange de n-dodécane et de n-tétradécane.

Selon un autre mode de réalisation particulier, on utilise un mélange n-dodécane : n-tétradécane (C₁₂/C₁₄) comprenant
a) de 65 à 95% en poids, de préférence de 70 à 90% en poids d'alcane linéaire volatil liquide en C₁₂ (n-dodécane) et
b) de 5 à 35% en poids, de préférence de 10 à 30% en poids, d'alcane linéaire liquide volatil en C₁₄ (n-tétradécane) par rapport au poids total des alcanes dans ledit mélange.

Le ou les alcanes volatils linéaires conformes à l'invention sont de préférence présents à des teneurs allant de 3 à 50% en poids et de préférence de 5 à 15% en poids par rapport au poids total de la composition.

### SUBSTANCES PARFUMANTES

Les parfums sont des compositions contenant notamment les matières premières decrites dans S. Arctander, Perfume and Flavor Chemicals (Montclair, N.J., 1969), dans S. Arctander, Perfume and Flavor Materials of Natural Origin (Elizabeth, N.J., 1960) et dans "Flavor and Fragrance Materials - 1991", Allured Publishing Co. Wheaton, III.

Il peut s'agir de produits naturels (huiles essentielles, absolus, résinoïdes, résines, concrètes) et/ou synthétiques (hydrocarbures terpéniques ou sesquiterpéniques, alcools, phénols, aldéhydes, cétones, éthers, acides, esters, nitriles, peroxydes, saturés ou insaturés, aliphatiques ou cycliques).

Selon la définition donnée dans la norme internationale ISO 9235 et adoptée par la Commission de la Pharmacopée Européenne, une huile essentielle est un produit odorant généralement de composition complexe, obtenu à partir d'une matière première végétale botaniquement définie, soit par entraînement à la vapeur d'eau, soit par distillation sèche, soit par un procédé mécanique approprié sans chauffage (Expression à froid). L'huile essentielle est le plus souvent séparée de la phase aqueuse par un procédé physique n'entraînant pas de changement significatif de la composition.

### Modes d'obtention des huiles essentielles

Le choix de la technique dépend principalement de la matière première : son état originel et ses caractéristiques, sa nature proprement dit. Le rendement « huile essentielle/matière première végétale » peut être extrêmement variable selon les plantes : 15 ppm à plus de 20%. Ce choix conditionne les caractéristiques de l'huile essentielle, en particulier viscosité, couleur, solubilité, volatilité, enrichissement ou appauvrissement en certains constituants.

### Entraînement à la vapeur d'eau

L'entrainement à la vapeur correspond à la vaporisation en présence de vapeur d'eau d'une substance peu miscible à l'eau. La matière première est mise en présence d'eau portée à ébullition ou de vapeur d'eau dans un alambic. La vapeur d'eau entraîne la vapeur d'huile essentielle qui est condensée dans le réfrigérant pour être récupérée en phase liquide dans un vase florentin (ou essencier) où l'huile essentielle est séparée de l'eau par décantation. On appelle « eau aromatique ou « hydrolat » ou « eau distillée florale », le distillat aqueux qui subsiste à l'entraînement à la vapeur d'eau, une fois la séparation de l'huile essentielle effectuée.

### Distillation sèche

L'huile essentielle est obtenue par distillation des bois, écorces ou racines, sans addition d'eau ou de vapeur d'eau dans une enceinte fermée conçue pour que le liquide soit récupéré dans sa partie basse. L'huile de Cade constitue l'exemple le plus connu de ce mode d'obtention.

### Expression à froid

Ce mode d'obtention ne s'applique qu'aux fruits agrumes (Citrus spp.) par des procédés mécaniques à température ambiante. Le principe de la méthode est le suivant : les zestes sont dilacérés et le contenu des poches sécrétrices qui ont été rompues est récupéré par un procédé physique. Le procédé classique consiste à exercer sous un courant d'eau une action abrasive sur toute la surface du fruit. Après élimination des déchets solides, l'huile essentielle est séparée de la phase aqueuse par centrifugation. La plupart des installations industrielles permettent en fait la récupération simultanée ou séquentielle des jus de fruits et de l'huile essentielle.

### Caractères physico-chimiques.

Les huiles essentielles sont en général volatiles et liquides à température ambiante, ce qui les différencie des huiles dites fixes. Elles sont plus ou moins colorées et leur densité est en général inférieure à celle de l'eau. Elles ont un indice de réfraction élevée et la plupart dévient la lumière polarisée. Elles sont liposolubles et solubles dans les solvants organiques usuels, entraînables à la vapeur d'eau, très peu solubles dans l'eau.

Parmi les huiles essentielles utilisables selon l'invention, on peut citer celles obtenues à partir des plantes appartenant aux familles botaniques suivantes :
Abiétaceés ou Pinacées : conifères
Amaryllidacées
Anacardiacées
Anonacées : ylang
Apiacées (par exemple les ombellifères) : aneth, angénique, coriandre, criste marine, carotte, persil
Aracées
Aristolochiacées
Astéracées : achilée, armoise, camomille, hélichryse
Bétulacées
Brassicacées
Burséracées : encens
Caryophyllacées
Canellacées
Césalpiniacées : copaïfera (copahu)
Chénopodacées
Cistacées : ciste
Cypéracées
Diptérocarpacées
Ericacées : gaulthérie (wintergreen)
Euphorbiacées
Fabacées
Geraniacées : géranium
Guttifères
Hamamélidacées
Hernandiacées
Hypéricacées : millepertuis
Iridacées
Juglandacées
Lamiacées : thym, origan, monarde, sarriette, basilic, marjolaines, menthes, patchouli, lavandes, sauges, cataire, romarin, hysope, mélisse, romarin
Lauracées : ravensara, laurier, bois de rose, cannelle, litséa
Liliacées : ail
Magnoliacées : magnolia
Malvacées
Méliacées
Monimiacées
Moracées : chanvre, houblon
Myricacées
Mysristicacées : muscade
Myrtacées : eucalyptus, tea tree, niaouli, cajeput, backousia, girofle, myrte
Oléacées
Pipéracées : poivre
Pittosporacées
Poacées : citronnelle, lemongrass, vétiver
Polygonacées
Renonculacées
Rosacées : roses
Rubiacées
Rutacées : tous les citrus
Salicacées
Santalacées : santal
Saxifragacées
Schisandracées
Styracacées : benjoin
Thymélacées : bois d'agar
Tilliacées
Valérianacées : valériane, nard
Verbénacées : lantana, verveine
Violacées
Zingibéracées : galanga, curcuma, cardamome, gingembre
Zygophyllacées

On peut citer également les huiles essentielles extraites de fleurs (lis, lavande, rose, jasmin, Ylang-Ylang, néroli), de tiges et de feuilles (patchouli, géranium, petit-grain), de fruits (coriandre, anis, cumin, genièvre), d'écorces de fruits (bergamote, citron, orange), de racines (angélique, céleri, cardamome, iris, acore, gingembre), de bois (bois de pin, santal, gaïac, cèdre rose, camphre), d'herbes et de graminées (estragon, romarin, basilic, lemon grass, sauge, thym), d'aiguilles et de branches (épicéa, sapin, pin, pin nain), de résines et de baumes (galbanum, élémi, benjoin, myrrhe, oliban, opopanax).

Des exemples de substances parfumantes sont notamment : le géraniol, l'acétate de géranyle, le farnésol, le bornéol, l'acétate de bornyle, le linalol, l'acétate de linalyle, le propionate de linalyle, le butyrate de linalyle, le tétrahydrolinalol, le citronellol, l'acétate de citronellyle, le formate de citronellyle, le propionate de citronellyle, le dihydromyrcenol, l'acétate de dihydromyrcenyle, le tétrahydromyrcenol, le terpinéol, l'acétate de terpinyle, le nopol, l'acétate de nopyle, le nérol, l'acétate de néryle, le 2-phényléthanol, l'acétate de 2-phényléthyle, l'alcool benzylique, l'acétate de benzyle, le salicylate de benzyle, l'acétate de styrallyle, le benzoate de benzyle, le salicylate d'amyle, le diméthylbenzyl-carbinol, l'acétate de trichlorométhylphénylcarbinyle, l'acétate de p-tert-butylcyclohexyle, l'acétate d'isononyle, l'acétate de vétivéryle, le vétivérol, l'alpha -hexylcinnamaldéhyde, le 2-méthyl-3-(p-tert-butylphényl)propanal, le 2-méthyl-3-(p-isopropylphényl)propanal, le 3-(p-tert-butylphényl)-propanal, le 2,4-diméthylcyclohex-3-enyl-carboxaldéhyde, l'acétate de tricyclodécènyle, le propionate de tricyclodécènyle, le 4-(4-hydroxy-4-méthylpentyl)-3-cyclohexènecarboxaldéhyde, le 4-(4-méthyl-3-pentènyl)-3-cyclohexènecarboxaldéhyde, le 4-acétoxy-3-pentyl-tétrahydropyrane, le 3-carboxyméthyl-2-pentylcyclopentane, la 2-n-4-heptylcyclopentanone, la 3-méthyl-2-pentyl-2-cyclopentènone, la menthone, la carvone, la tagétone, la géranyl acétone, le n-décanal, le n-dodécanal, le 9-décènol-1, l'isobutyrate de phénoxyéthyle, le phényl-acétaldéhyde diméthyl-acétal, le phénylacétaldéhyde diéthylacétal, le géranonitrile, le citronellonitrile, l'acétate de cédryle, le 3-isocamphylcyclohexanol, le cédryl méthyl éther, l'isolongifolanone, l'aubépinonitrile, l'aubépine, l'héliotropine, la coumarine, l'eugénol, la vanilline, l'oxyde de diphényle, le citral, le citronellal, l'hydroxycitronellal, la damascone, les ionones, les méthylionones, les isométhylionones, la solanone, les irones, le cis-3-hexènol et ses esters, les muscs-indanes, les muscs-tétralines, les muscs-isochromanes, les cétones macrocycliques, les muscs-macrolactones, les muscs aliphatiques, le brassylate d'éthylène et leurs mélanges.

Selon un mode préféré de réalisation de l'invention, on utilise un mélange de différentes substances parfumantes qui engendrent en commun une note plaisante pour l'utilisateur.

On choisira de préférence les substances parfumantes de telles sorte qu'elle produisent des notes (tête, coeur et fond) dans les familles suivantes
les hespéridés,
les aromatiques,
les notes florales
les épicées,
les boisées
les gourmands
les chyprés,
les fougères
les cuirés.
les muscs

Les compositions parfumantes de l'invention contiennent de préférence de 2% à 40 % en poids de substance parfumante, mieux de 2% à 30% en poids, en particulier de 2% à 20% en poids par rapport au poids total de la composition.

### FORMES GALENIQUES

Les compositions parfumantes peuvent se présenter sous toutes les formes galéniques aqueuses à usage topique normalement utilisées et être notamment sous forme d'émulsions obtenues par dispersion d'une phase grasse dans une phase aqueuse (H/E) ou inversement (E/H), d'émulsions triples (E/H/E ou H/E/H) ou de dispersions vésiculaires de type ionique et/ou non ionique. Ces compositions sont préparées selon les méthodes usuelles. La composition selon l'invention se présente sous forme d'une émulsion H/E.

En outre, les compositions selon l'invention peuvent être plus ou moins fluides et avoir l'aspect d'une crème, d'une pommade, d'un lait, d'une pâte, d'une mousse. Elles peuvent aussi se présenter sous forme solide, et par exemple sous forme de stick.

Elle peut constituer une composition de parfumage, de soin, de traitement des matières kératiniques, et notamment se présenter sous forme de lait, crème pommade, baume de soin ; de produit d'hygiène corporelle comme des produits déodorants, des gels douches, des produits pour le bain, des shampooings, des scrubs. Elle peut également se présenter sous la forme d'une lotion multi-phasique parfumante notamment une lotion bi-phasique.

Elle peut être conditionnée dans un flacon, dans un pot, dans un tube, sous forme roll-on, de bouillotte ou de lingettes.

La composition parfumante de l'invention peut être diffusée selon différents systèmes comme des sprays, des aérosols, des dispositifs piézoélectriques.

La composition parfumante selon l'invention peut être fabriquée par les procédés connus, généralement utilisés dans le domaine des formulations parfumées.

Les compositions parfumantes selon l'invention peuvent également être appliquées sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple le diméthyléther, l'isobutane, le n-butane, le propane, le trichlorofluorométhane.

La teneur en eau de la composition ira de préférence de 40 à 70% en poids par rapport au poids total de la composition.

Lorsque la composition est une émulsion, la proportion de la phase grasse peut aller de 5 à 80 % en poids, et de préférence de 5 à 50 % en poids par rapport au poids total de la composition.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant la phase continue de l'émulsion à obtenir (E/H ou H/E). Quand l'émulsion est multiple, elle comporte généralement un émulsionnant dans l'émulsion primaire et un émulsionnant dans la phase externe dans laquelle est introduite l'émulsion primaire.

Comme émulsionnants utilisables pour la préparation des émulsions E/H, on peut citer par exemple les alkyl esters ou éthers de sorbitan, de glycérol ou de sucres ; les tensioactifs siliconés comme les dimethicone copolyols tels que le mélange de cyclomethicone et de dimethicone copolyol, vendu sous les dénominations DC 5225 C et DC 3225 C par la société Dow Corning, et comme les alkyl-dimethicone copolyols tels que le Laurylmethicone copolyol vendu sous la dénomination "Dow Corning 5200 Formulation Aid" par la société Dow Corning, le Cetyl dimethicone copolyol vendu sous la dénomination Abil EM 90R par la société EVONIK et le mélange de Polyglyceryl-4 isostearate/Cetyl dimethicone copolyol/Hexyl laurate vendu sous la dénomination Abil WE 09R par la société EVONIK. On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan GI 34 par la société EVONIK, l'isostéarate de sorbitan, tel que le produit commercialisé sous la dénomination Arlacel 987 par la société CRODA, l'isostéarate de sorbitan et de glycérol, tel que le produit commercialisé sous la dénomination Arlacel 986 par la société CRODA, et leurs mélanges.

Comme émulsionnants utilisables pour la préparation des émulsions H/E, on peut citer par exemple les émulsionnants non ioniques tels que les esters d'acides gras et de polyols oxyalkylénés (plus particulièrement polyoxyéthylénés), et par exemple les stéarates de polyéthylène glycol comme le stéarate de PEG-100, le stéarate de PEG-50 et le stéarate de PEG-40 ; et leurs mélanges tels que le mélange de mono-stéarate de glycéryle et de stéarate de polyéthylène glycol (100 OE) commercialisé sous la dénomination SIMULSOL 165 par la société SEPPIC ; les esters d'acides gras et de sorbitan oxyalkylénés comprenant par exemple de 20 à 100 OE, et par exemple ceux commercialisés sous les dénominations commerciales Tween 20 ou Tween 60 par la société CRODA ; les éthers d'alcools gras oxyalkylénés (oxyéthylénés et/ou oxypropylénés) ; les esters de sucres comme le stéarate de sucrose ; et leurs mélanges comme par exemple le mélange de stéarate de glycéryle et de stéarate de PEG-100, commercialisé sous la dénomination Arlacel 165 par la société CRODA.

On peut ajouter à ces émulsionnants, des co-émulsionnants tels que par exemple les alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique.

On peut aussi préparer des émulsions sans tensioactifs émulsionnants ou en contenant moins de 0,5 % du poids total de la composition, en utilisant des composés appropriés, par exemple les polymères ayant des propriétés émulsionnantes tels que les polymères commercialisés sous les dénominations Carbopol 1342 et Pemulen par la société LUBRIZOL ; ou les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination « Hostacerin AMPS » (nom CTFA : ammonium polyacryldimethyltauramide) ou comme le polymère en émulsion commercialisé sous la dénomination Sepigel 305 par la société Seppic (nom INCI : Polyacrylamide / C13-C14 isoparaffine / laureth-7) ; les particules de polymères ioniques ou non ioniques, plus particulièrement des particules de polymère anionique comme notamment les polymères d'acide isophtalique ou d'acide sulfoisophtalique, et en particulier les copolymères de phtalate / sulfoisophtalate / glycol (par exemple diéthylèneglycol / Phtalate / isophtalate/1,4-cyclohexane-diméthanol (nom INCI : Diglycol/ CHDM/I sophtalates/ SIP Copolymer) vendus sous les dénominations Eastman AQ polymer (AQ35S, AQ38S, AQ55S, AQ48 Ultra) par la société Eastman Chemical.

On peut aussi préparer des émulsions sans émulsionnants, stabilisées par des particules siliconées ou des particules d'oxyde métallique tels que TiO2 ou autres.

### ADDITIFS

La composition de l'invention peut comprendre, en outre, tout additif usuellement utilisé dans le domaine des parfums choisi notamment parmi les antioxydants ; des corps gras comme des huiles ou des cires ; les actifs cosmétiques ou dermatologiques comme par exemple les émollients ou adoucissants comme les céramides, les huiles d'amande douce, de noyau d'abricot ; les agents hydratants comme les hydrolysats de protéines, les polyols tels que la glycérine, les glycols et les dérivés de sucre, les hydroxyacides ; de l'eau déminéralisée et/ou une eau florale telle que de l'eau de rose, de l'eau de bleuet, de l'eau de camomille ou de l'eau de tilleul, et/ou une eau thermale ou minérale naturelle comme par exemple l'Eau de La Roche Posay ou l'Eau de Vichy ; les agents apaisants comme l'a-bisabolol, l'allantoïne, aloes vera ; les vitamines ; les acides gras essentiels ; les agents répulsifs contre les insectes ; les propulseurs ; les peptisants ; des charges ; des co-solvants ; des filtres UV ; des stabilisants, bactéricides ou conservateurs ; des agents structurants ; des gélifiants ou épaississants hydrophiles ou lipophiles; des colorants ; des nacres ; des paillettes ; des électrolytes tel que le chlorure de sodium, phosphate de sodium ; des ajusteurs de pH comme par exemple acide citrique ou hydroxyde de sodium), et leurs mélanges.

Parmi les antioxydants, on peut citer par exemple le BHA (tert-butyl-4-hydroxyanisole), le BHT (2,6-di-tert-butyl-p-crésol), les tocophérols comme la vitamine E et ses dérivés tels que l'acétate de tocophéryle.

Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société ARKEMA ; les poudres de polyéthylène ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les micro-sphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition.

La composition selon l'invention peut notamment comprendre au moins une matière colorante comme les matières pulvérulentes, les colorants liposolubles, les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants solubles sont par exemple : le caramel, Yellow 5 , Acid Blue 9/ Blue 1, Green 5, Green 3 / Fast Green FCF 3, Orange 4, Red 4 / Food Red 1, Yellow 6, Acid Red 33 / Food Red 12, Red 40, le carmin de cochenille (CI 15850, CI 75470), Ext. Violet 2, Red 6-7, Ferric Ferrocyanide, Ultramarines, Acide Yellow 3 / Yellow 10, Acid Blue 3, Yellow 10.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.

Comme gélifiants utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium ou encore les copolymères d'alcènes.

Comme huiles ou cires utilisables dans l'invention, on peut citer les huiles minérales (huile de vaseline), les huiles d'origine végétale (fraction liquide du beurre de karité, huile de tournesol), les huiles d'origine animale (perhydrosqualène), les huiles de synthèse (myristate d'isopropyle), les huiles ou cires de silicone (cyclométhicone) et les huiles fluorées (perfluoropolyéthers), les cires d'abeille, de carnauba ou paraffine. On peut ajouter à ces huiles des alcools gras (alcool cétylique) et des acides gras (acide stéarique).

Parmi les co-solvants utilisables selon l'invention, on peut citer l'éthanol ou isopropanol, l'octyldodécanol, le tréthylcitrate, le dicaprylylcarbonate, l'isononanoate d'isononyle, le myristate et palmitate d'ispropyle, le palmitate de 2-éthylhexyle.

L'invention va maintenant être décrite en référence aux exemples suivants donnés à titre illustratif et non limitatif. Dans ces exemples, sauf indication contraire, les quantités sont exprimées en pourcentages pondéraux. On a réalisé les formulations parfumées suivantes ; les quantités sont indiquées en pourcentages en poids :

### Etude comparative du profil d'évaporation

### Points d'Eclair :

La matière première est chauffée dans une coupe fermée de dimensions normalisées à une température d'environ 3°C inférieure au point éclair supposé, pendant 60 secondes. Puis une flamme de dimension normalisée est présentée dans les vapeurs de la coupe par une ouverture coulissante. L'essai est répété de 1°C en 1°C. La plus basse température à laquelle se produit l'inflammation est notée comme étant le point éclair. L'essai est réalisé sur un appareil SETAFLASH selon la norme ISO 3679.

n-dodécane (invention) : 71°C
Mélange undécane/tridécane (invention) selon l'exemple 1
ou l'exemple 2 du WO2008/155059 : 81°C
Dicaprylyl carbonate : >200°C
Dicaprylyl ether : 138°C
Huile de Colza : >200°C
Isododecane : 43°C
Ethanol : 13°C

On note rapidement que les points éclairs des différents composés hydrocarbonés sont bien plus élevés que celui de l'éthanol. Il est donc plus facile de travailler ces matières premières à chaud notamment dans le cas d'émulsion dont le procédé de fabrication est supérieur à 50°C.

### Evaporation :

On réalise les compositions cosmétiques suivantes ; puis on mesure le pourcentage d'évaporation de chacune des formulations à l'aide d'une thermobalance à infrarouge METLER TOLEDO HR 83 Halogen à une température de 105°C :

| **Ingrédients** | **Ex1 (*)** | **Ex2 (*)** | **EX3 (*)** | **EX4** | **EX5** | **EX6(*)** |
|---|---|---|---|---|---|---|
| Copolymère réticulé d'acide acrylamido 2-méthyl propane sulfonique et d'ester d'acide (méth)acrylique et d'alcool gras en C₁₆-C₁₈ polyoxyéthyléné à 25 OE (GENAPOL T-250) tel que celui décrit dans l'exemple 3 de la demande EP1059142 | 0,80% | 0,80% | 0,80% | 0,80% | 0,80% | 0,80% |
| Conservateur | 0,20% | 0,20% | 0,20% | 0,20% | 0,20% | 0,20% |
| Parfum Mennen Green Tonic After Shave | 2% | 2% | 2% | 2% | 2% | 2% |
| Huile végétale de colza | 10% | - | - | - | - | - |
| Dicaprylyl ether (CETIOL OE) | - | 10% | - | - | - | - |
| Dicaprylyl carbonate (CETIOL CC) | - | - | 10% | - | - | - |
| n-dodécane | - | - | - | 10% | - | - |
| Mélange Undécane/Tridécane selon l'exemple 1 ou l'exemple 2 du WO2008/155059 | - | - | - | - | 10% | - |
| Ethanol | - | - | - | - | - | 10% |
| Eau déminéralisée | qsp 100% | qsp 100% | qsp 100% | qsp 100% | qsp 100% | qsp 100% |

| | | | | | | |
|---|---|---|---|---|---|---|
| (*) hors invention | | | | | | |

### Résultats :

| **Ingrédients** | **Ex1 (*)** | **Ex2 (*)** | **EX3 (*)** | **EX4** | **EX5** | **EX6(*)** |
|---|---|---|---|---|---|---|
| Extrait sec à 105°C | 11,03% | 11,97% | 11,28% | 2,19% | 2,11% | 1,87% |
| Pourcentage d'évaporation | 88.97% | 88.03% | 88.72% | 97.81% | 97.89% | 98.13% |

| | | | | | | |
|---|---|---|---|---|---|---|
| (*) hors invention | | | | | | |

Les mesures effectuées montrent que les exemples 1 à 3 de composition parfumée contenant soit un ester, un éther ou encore une huile végétale ont un profil d'évaporation faible.

En revanche les exemples 4 et 5 de l'invention contenant des alcanes linéaires volatils comme le n-dodécane et le mélange undécane/tridécane présentent un bien meilleur profil d'évaporation et de diffusion du parfum qui est comparable à celui de l'exemple 7 avec l'éthanol ou à celui de l'exemple 6 sans composé hydrocarboné liquide dont l'évaporation est maximale.

### Exemple 7 : Déodorant crème :

| | |
|---|---|
| Mélange Undecane/Tridecane selon l'exemple 1 ou l'exemple 2 du document WO2008/155059 | 10% |
| Steareth-2 | 3% |
| Steareth-21 | 2% |
| PPG-15 Stearyl Ether | 6% |
| Aluminium Chlorhydrate | 15% |
| Parfum Mennen Green Tonic After Shave | 3% |
| Eau déminéralisée | qsp 100% |

### Exemple 8 : Lait pour le corps

| | |
|---|---|
| PEG-100 Stearate / Glyceryl stearate | 5% |
| Carbomer | 0.5% |
| Triéthanolamine | 0.5% |
| Triglycérides en C₈-C₁₀ | 12% |
| Octyldodécanol | 5% |
| Cetyl alcohol | 4% |
| Dimethicone | 2% |
| n-dodécane | 10% |
| Parfum Mennen Green Tonic After Shave | 3% |
| Eau déminéralisée | qsp100% |

## Revendications

1. Procédé de parfumage des matières kératiniques ou d'un vêtement **caractérisé par le fait qu'**il consiste à appliquer sur lesdites matières kératiniques ou ledit vêtement une composition parfumante aqueuse sous forme d'émulsion huile-dans-eau comprenant dans un milieu cosmétiquement acceptable
a) de 30 à 80% en poids d'eau par rapport au poids total de la composition ;
b) au moins 2% en poids d'une substance parfumante par rapport au poids total de la composition ;
c) au moins un alcane linéaire volatil ou un mélange d'alcanes linéaires volatils, ledit ou lesdits alcanes volatils comprenant de 7 à 14 atomes de carbone.

2. Procédé selon la revendication 1, dans lequel le ou les alcanes volatils comprennent de 8 à 14 atomes de carbone, plus préférentiellement de 11 à 14 atomes de carbone.

3. Procédé selon la revendication 1 ou 2, dans lequel le ou les alcanes linéaires volatils présentent une vitesse d'évaporation à température ambiante (25°C) et pression atmosphérique (760 mm Hg) allant de 0,01 à 0,8 mg/cm²/min, de préférence allant de 0,01 à 0,3 mg/cm²/min et plus préférentiellement allant de 0,01 à 0,12 mg/cm²/min.

4. Procédé selon l'une quelconque des revendications 1 à 3, dans lequel le ou les alcanes linéaires volatils sont d'origine végétale.

5. Procédé selon l'une quelconque des revendications 1 à 4, dans lequel le ou les alcanes linéaires volatils sont choisis parmi le n- heptane (C₇), le n-octane (C₈), le n-nonane (C₉), le n-décane (C₁₀), le n-undécane (C₁₁), le n-dodécane (C₁₂), le n-tridécane (C₁₃), le n-tétradecane (C₁₄), et leurs mélanges.

6. Procédé selon l'une quelconque des revendications 1 à 5, dans lequel la composition comprend au moins deux alcanes linéaires liquides volatils distincts, différant entre eux d'un nombre de carbone n d'au moins 1, en particulier différant entre eux d'un nombre de carbone de 1 ou de 2.

7. Procédé selon l'une quelconque des revendications 1 à 6, dans lequel l'alcane linéaire volatil ou le mélange d'alcanes linéaires volatils comprend au moins un isotope ¹⁴C du carbone (carbone 14), en particulier l'isotope ¹⁴C peut être présent en un ratio ¹⁴C / ¹²C supérieur ou égal à 1.10⁻¹⁶, de préférence supérieur ou égal à 1.10⁻¹⁵, de préférence encore supérieur ou égal 7,5.10⁻¹⁴, et mieux supérieur ou égal à 1,5.10⁻¹³.

8. Procédé selon l'une quelconque des revendications 1 à 7, dans lequel la composition comprend un mélange d'au moins deux alcanes linéaires volatiles comprenant
a) de 50 à 90% en poids du mélange, de préférence de 55 à 80% en poids, préférentiellement encore de 60 à 75% en poids, d'un alcane linéaire liquide volatil en Cₙ, et
b) de 10 à 50% en poids du mélange, de préférence de 20 à 45% en poids, de préférence de 24 à 40% en poids, d'un alcane linéaire liquide volatil en Cₙ₊ₓ avec x supérieur ou égal à 1 et de préférence x=1 ou x=2, par rapport au poids total des alcanes dans ledit mélange.

9. Procédé selon l'une quelconque des revendications 1 à 8, dans lequel la composirion comprend un mélange de n-undécane (C₁₁) et de n-tridécane (C₁₃).

10. Procédé selon la revendication 9, dans lequel la composition comprend un mélange n-undécane : n-tridécane (C₁₁/C₁₃) comprenant
a) de 55 à 80% en poids, de préférence de 60 à 75% en poids d'alcane linéaire liquide volatil en C₁₁ (n-undécane), et
b) de 20 à 45% en poids, de préférence de 24 à 40% en poids, d'alcane linéaire liquide volatil en C₁₃ (n-tridécane) par rapport au poids total des alcanes dans ledit mél

11. Procédé selon l'une quelconque des revendications 1 à 8, dans lequel l'alcane linéaire volatil est choisi parmi le n-dodécane (C₁₂), le n-tétradécane (C₁₄) ou leurs mélanges.

12. Procédé selon la revendication 11, dans lequel la composition comprend un mélange n-dodécane : n-tétradécane (C₁₂/C₁₄) comprenant
a) de 65 à 95% en poids, de préférence de 70 à 90% en poids d'alcane linéaire volatil liquide en C₁₂ (n-dodécane) et
b) de 5 à 35% en poids, de préférence de 10 à 30% en poids, d'alcane linéaire liquide volatil en C₁₄ (n-tétradécane) par rapport au poids total des alcanes dans ledit mélange.

13. Procédé selon l'une quelconque des revendications 1 à 12, dans lequel la teneur en eau dans la composition varie de 40 à 70% en poids par rapport au poids total de la composition.

## Patentansprüche

1. Verfahren zum Parfümieren von Keratinmaterialien oder eines Kleidungsstücks,
**dadurch gekennzeichnet, dass** dieses daraus besteht, auf die Keratinmaterialien oder das Kleidungsstück eine parfümierende wässrige Zusammensetzung in der Form einer Öl-in-Wasser-Emulsion aufzubringen, umfassend in einem kosmetisch annehmbaren Medium
a) von 30 bis 80 Gew.-% Wasser im Verhältnis zum Gesamtgewicht der Zusammensetzung;
b) mindestens 2 Gew.-% einer parfümierenden Substanz im Verhältnis zum Gesamtgewicht der Zusammensetzung;
c) mindestens ein flüchtiges lineares Alkan oder eine Mischung flüchtiger linearer Alkane, wobei das oder die flüchtigen Alkane von 7 bis 14 Kohlenstoffatome umfassen.

2. Verfahren nach Anspruch 1,
wobei das oder die flüchtigen Alkane von 8 bis 14 Kohlenstoffatome, bevorzugter von 11 bis 14 Kohlenstoffatome, umfassen.

3. Verfahren nach Anspruch 1 oder 2,
wobei das oder die flüchtigen linearen Alkane eine Verdampfungsgeschwindigkeit bei Umgebungstemperatur (25°C) und Atmosphärendruck (760 mm Hg) von 0,01 bis 0,8 mg/cm²/min aufweisen, vorzugsweise von 0,01 bis 0,3 mg/cm²/min und bevorzugter von 0,01 bis 0,12 mg/cm²/min.

4. Verfahren nach einem der Ansprüche 1 bis 3, wobei das oder die flüchtigen linearen Alkane pflanzlichen Ursprungs sind.

5. Verfahren nach einem der Ansprüche 1 bis 4, wobei das oder die flüchtigen linearen Alkane ausgewählt werden aus n-Heptan (C₇), n-Octan (C₈), n-Nonan (C₉), n-Decan (C₁₀), n-Undecan (C₁₁), n-Dodecan (C₁₂), n-Tridecan (C₁₃), n-Tetradecan (C₁₄) und ihren Mischungen.

6. Verfahren nach einem der Ansprüche 1 bis 5, wobei die Zusammensetzung mindestens zwei verschiedene flüchtige flüssige lineare Alkane umfasst, die sich voneinander in einer Kohlenstoffzahl n von mindestens 1 unterscheiden, insbesondere sich voneinander in der Kohlenstoffzahl von 1 oder 2 unterscheiden.

7. Verfahren nach einem der Ansprüche 1 bis 6, wobei das flüchtige lineare Alkan oder die Mischung von flüchtigen linearen Alkanen mindestens ein Isotop ¹⁴C (Kohlenstoff 14) umfasst, wobei das Isotop ¹⁴C insbesondere in einem Verhältnis ¹⁴C/¹²C größer oder gleich 1.10⁻¹⁶ vorliegen kann, vorzugsweise größer oder gleich 1.10⁻¹⁵, vorzugsweise auch größer oder gleich 7,5.10⁻¹⁴, und besser größer oder gleich 1,5.10⁻¹³.

8. Verfahren nach einem der Ansprüche 1 bis 7, wobei die Zusammensetzung eine Mischung von mindestens zwei flüchtigen linearen Alkanen umfasst, umfassend:
a) von 50 bis 90 Gew.-% der Mischung, vorzugsweise von 55 bis 80 Gew.-%, auch bevorzugt von 60 bis 75 Gew.-%, eines flüchtigen flüssigen linearen Cₙ-Alkans, und
b) von 10 bis 50 Gew.-% der Mischung, vorzugsweise von 20 bis 45 Gew.-%, vorzugsweise von 24 bis 40 Gew.-%, eines flüchtigen flüssigen linearen Cₙ₊ₓ-Alkans, wobei x größer oder gleich 1 ist, und vorzugsweise x=1 oder x=2, im Verhältnis zum Gesamtgewicht der Alkane der Mischung.

9. Verfahren nach einem der Ansprüche 1 bis 8,
wobei die Zusammensetzung eine Mischung von n-Undecan (C₁₁) und von n-Tridecan (C₁₃) umfasst.

10. Verfahren nach Anspruch 9,
wobei die Zusammensetzung eine Mischung n-Undecan:n-Tridecan (C₁₁/C₁₃) umfasst, umfassend
a) von 55 bis 80 Gew.-%, vorzugsweise von 60 bis 75 Gew.-%, eines flüchtigen flüssigen linearen C₁₁-Alkans (n-Undecan), und
b) von 20 bis 45 Gew.-%, vorzugsweise von 24 bis 40 Gew.-%, eines flüchtigen flüssigen linearen C₁₃-Alkans (n-Tridecan) im Verhältnis zum Gesamtgewicht der Alkane in der Mischung.

11. Verfahren nach einem der Ansprüche 1 bis 8,
wobei das flüchtige lineare Alkan ausgewählt wird aus n-Dodecan (C₁₂), n-Tetradecan (C₁₄) oder ihren Mischungen.

12. Verfahren nach Anspruch 11,
wobei die Zusammensetzung eine Mischung n-Dodecan:n-Tetradecan (C₁₂/C₁₄) umfasst, umfassend
a) von 65 bis 95 Gew.-%, vorzugsweise von 70 bis 90 Gew.-%, eines flüchtigen flüssigen linearen C₁₂-Alkans (n-Dodecan), und
b) von 5 bis 35 Gew.-%, vorzugsweise von 10 bis 30 Gew.-%, eines flüchtigen flüssigen linearen C₁₄-Alkans (n-Tetradecan) im Verhältnis zum Gesamtgewicht der Alkane in der Mischung.

13. Verfahren nach einem der Ansprüche 1 bis 12, wobei der Wassergehalt in der Zusammensetzung von 40 bis 70 Gew.-% im Verhältnis zum Gesamtgewicht der Zusammensetzung variiert.

## Claims

1. Process for fragrancing keratin materials or clothing, **characterized in that** it consists in applying to the said keratin materials or the said clothing an aqueous fragrancing composition in the form of an oil-in-water emulsion comprising, in a cosmetically acceptable medium:
a) from 30 to 80% by weight of water relative to the total weight of the composition;
b) at least 2% by weight of a fragrancing substance relative to the total weight of the composition;
c) at least one volatile linear alkane or a mixture of volatile linear alkanes, said volatile alkane(s) comprising from 7 to 14 carbon atoms.

2. Process according to Claim 1, in which the volatile alkane(s) comprise from 8 to 14 carbon atoms and more preferentially from 11 to 14 carbon atoms.

3. Process according to Claim 1 or 2, in which the volatile linear alkane(s) have an evaporation rate at room temperature (25°C) and atmospheric pressure (760 mmHg) ranging from 0.01 to 0.8 mg/cm²/minute, preferably ranging from 0.01 to 0.3 mg/cm²/minute and more preferentially ranging from 0.01 to 0.12 mg/cm³/minute.

4. Process according to any one of Claims 1 to 3, in which the volatile linear alkane (s) are of plant origin.

5. Process according to any one of Claims 1 to 4, in which the volatile linear alkane(s) are chosen from n-heptane (C₇), n-octane (C₈), n-nonane (C₉), n-decane (C₁₀), n-undecane (C₁₁), n-dodecane (C₁₂), n-tridecane (C₁₃) and n-tetradecane (C₁₄), and mixtures thereof.

6. Process according to any one of Claims 1 to 5, wherein the composition comprises at least two different volatile liquid linear alkanes, differing from each other by a carbon number n of at least 1, in particular differing from each other by a carbon number of 1 or 2.

7. Process according to any one of Claims 1 to 6, in which the volatile linear alkane or the mixture of volatile linear alkanes comprises at least one ¹⁴C (carbon-14) carbon isotope, and in particular the ¹⁴C isotope may be present in a ¹⁴C/¹²C ratio of greater than or equal to 1×10⁻¹⁶, preferably greater than or equal to 1×10⁻¹⁵, more preferably greater than or equal to 7.5×10⁻¹⁴ and better still greater than or equal to 1.5×10⁻¹³.

8. Process according to any one of Claims 1 to 7, wherein the composition comprises a mixture of at least two volatile linear alkanes comprising:
a) from 50% to 90% by weight of the mixture, preferably from 55% to 80% by weight and more preferentially from 60% to 75% by weight, of a Cₙ volatile liquid linear alkane, and
b) from 10% to 50% by weight of the mixture, preferably from 20% to 45% by weight and preferably from 24% to 40% by weight, of a Cₙ₊ₓ volatile liquid linear alkane with x greater than or equal to 1 and preferably x = 1 or x = 2, relative to the total weight of alkanes in the said mixture.

9. Process according to any one of Claims 1 to 8, wherein the composition comprises a mixture of n-undecane (C₁₁) and of n-tridecane (C₁₃) .

10. Process according to Claim 9, wherein the composition comprises an n-undecane/n-tridecane (C₁₁/C₁₃) mixture comprising:
a) from 55% to 80% by weight and preferably from 60% to 75% by weight of C₁₁ volatile liquid linear alkane (n-undecane), and
b) from 20% to 45% by weight and preferably from 24% to 40% by weight of C₁₃ volatile liquid linear alkane (n-tridecane) relative to the total weight of alkanes in the said mixture.

11. Process according to any one of Claims 1 to 8, in which the volatile linear alkane is chosen from n-dodecane (C₁₂) and n-tetradecane (C₁₄), or mixtures thereof.

12. Process according to Claim 11, wherein the composition comprises an n-dodecane/n-tetradecane (C_{12/}C₁₄) mixture comprising:
a) from 65% to 95% by weight and preferably from 70% to 90% by weight of C₁₂ liquid volatile linear alkane (n-dodecane) and
b) 5% to 35% by weight and preferably from 10% to 30% by weight of C₁₄ volatile liquid linear alkane (n-tetradecane) relative to the total weight of alkanes in the said mixture.

13. Process according to any one of Claims 1 to 12, wherein the water content in the composition ranges from 40% to 70% by weight relative to the total weight of the composition.
